(19) Europäisches Patentamt
European Patent Office
Office européen des brevets

(11) Veröffentlichungsnummer: **0 292 906 A2**

(12) # EUROPÄISCHE PATENTANMELDUNG

(21) Anmeldenummer: **88108200.2**

(51) Int. Cl.[4]: **C09B 62/503 , C09B 19/02**

(22) Anmeldetag: **21.05.88**

(30) Priorität: **27.05.87 DE 3717832**

(43) Veröffentlichungstag der Anmeldung:
**30.11.88 Patentblatt 88/48**

(84) Benannte Vertragsstaaten:
**BE CH DE FR GB IT LI**

(71) Anmelder: **HOECHST AKTIENGESELLSCHAFT**
**Postfach 80 03 20**
**D-6230 Frankfurt am Main 80(DE)**

(72) Erfinder: **Helmling, Walter, Dr.**
**Fichtestrasse 29**
**D-6238 Hofheim am Taunus(DE)**
Erfinder: **Hähnle, Reinhard, Dr..**
**Kastanienweg 7a**
**D-6240 Königstein/Taunus(DE)**

(54) **Verfahren zur Herstellung von Triphendioxazinverbindungen.**

(57) Herstellung von anionischen Triphendioxazinverbindungen durch Cyclisierung der entsprechenden 2,5-Di-(arylamino)-1,4-benzochinon-Verbindungen in Oleum oder 95 bis 100%iger Schwefelsäure als Cyclisierungsmedium, bei welchem Wasserstoffperoxid als oxidierendes Cyclisierungshilfsmittel eingesetzt und verwendet wird.

EP 0 292 906 A2

Xerox Copy Centre

## Verfahren zur Herstellung von Triphendioxazinverbindungen

Die Erfindung liegt auf dem technischen Gebiet der anionischen Triphendioxazinfarbstoffe.

Es ist seit langem bekannt (s. beispielsweise H.R. Schweizer, Künstliche organische Farbstoffe und ihre Zwischenprodukte, Springer Verlag, 1964, Seiten 283 u. 284), anionische Triphendioxazinverbindungen durch Cyclisierungsreaktion von 2,5-Di-(arylamino)-1,4-benzochinonen, die in der Regel durch Umsetzung eines halogensubstituierten 1,4-Benzochinons, wie 2,3,5,6-Tetrachlor-1,4-benzochinon (Chloranil), mit der zweifach molaren Menge einer Arylamino-Verbindung hergestellt werden, unter Verwendung von Kondensationsmitteln, wie Schwefelsäure und Schwefeltrioxid enthaltender Schwefelsäure (Oleum), herzustellen. Bei dieser Verfahrensweise wird häufig beobachtet, daß insbesondere bei solchen Di-(arylamino)-benzochinon-Ausgangsverbindungen, die im Arylrest des Arylamins Sulfonsäure- oder Sulfonylgruppen enthalten, die Cyclisierungsreaktion schwierig durchzuführen ist und daß höhere Reaktionstemperaturen und der Einsatz von höherprozentigem Oleum erforderlich ist, weswegen unerwünschte Nebenreaktionen, wie intra- oder intermolekulare Dehydratisierungsreaktionen, Kondensationsreaktionen, Hydrolyse oder oxidativer Abbau, auftreten können und demzufolge uneinheitliche Endprodukte gebildet werden, wenn die Herstellung der gewünschten Triphendioxazinverbindungen nicht gar überhaupt verhindert wird.

Eine Verbesserung dieser bekannten Verfahrensweise erfolgte durch die zusätzliche Verwendung von salzförmigen anorganischen Oxidationsmitteln, wie beispielsweise Natrium-, Kalium oder Ammoniumperoxodisulfat (s. britische Patentschrift Nr. 1 589 915), oder von anderen Oxidationsmitteln, wie Jod oder anorganischen Jodverbindungen (s. Europäische Patentanmeldungs-Veröffentlichung Nr. 0 141 359A), oder Mangandioxid (s. deutsche Offenlegungsschrift Nr. 3 510 613A).

Diese Verfahrensweisen haben jedoch den Nachteil, daß die bei der Aufarbeitung als Mutterlauge anfallende verdünnte Schwefelsäure mit anorganischen Salzen verunreinigt ist, die durch die bei der Cyclisierung verwendeten anorganischen Oxidationsmittel eingebracht werden. Dies führt dazu, daß die Weiterverwendung der Abfallschwefelsäure bzw. ihre Aufarbeitung oder Aufkonzentrierung stark eingeschränkt ist und daß der Einsatz spezieller, technisch besonders vorteilhafter Aufarbeitungsprozesse (Spaltprozesse), wie sie beispielsweise in der Zeitschrift Chem. Ing. Tech. 50 (1978), 30 ff., insbes. 32-34, beschrieben sind, sogar unmöglich gemacht werden.

Da die Aufarbeitung von Abfallschwefelsäure oder deren Weiterverwendung schon aus Umweltgründen unbedingt erforderlich ist, stellte sich die Aufgabe, bei den üblichen Herstellungsverfahren für Triphendioxazinverbindungen durch Cyclisierungsreaktion in Schwefelsäure oder Oleum ein anderes, gleichwirkendes Oxidationshilfsmittel zu finden, das die Schwefelsäure jedoch nicht nachteilig verunreinigt.

Es wurde nun gefunden, daß man die Cyclisierungsreaktion von 2,5-Di-(arylamino)-benzochinon-Verbindungen in Schwefelsäure, bevorzugt in Oleum, ohne die angegebenen Probleme durchführen kann, wenn man als zusätzliches oxidatives Cyclisierungshilfsmittel Wasserstoffperoxid einsetzt, d.h. die Cyclisierung in wasserstoffperoxidhaltiger Schwefelsäure, bevorzugt wasserstoffperoxidhaltigem Oleum, durchführt.

Die Erfindung betrifft somit ein Verfahren zur Herstellung von anionischen Triphendioxazinverbindungen, insbesondere von solchen, die in den Phenresten Sulfonsäuregruppen und/oder Sulfonylgruppen, wie beispielsweise die faserreaktiven Gruppen aus der Vinylsulfonreihe oder andere gegebenenfalls substituierte ($C_1$-$C_6$)-Alkylsulfonylgruppen oder gegebenenfalls substituierte Sulfonamidgruppen, enthalten, durch Cyclisierungsreaktion von 2,5-Di-(arylamino)-1,4-benzochinon-Verbindungen, wobei deren Arylreste weitere Substituenten enthalten können, wie insbesondere substituierte Aminogruppen und die erwähnten Sulfonsäure- und Sulfonylgruppen, und wobei die Arylreste bevorzugt Phenylreste sind, in Oleum oder 95 bis 100 %iger Schwefelsäure als Cyclisierungsmedium, das dadurch gekennzeichnet ist, daß man als oxidierendes Cyclisierungshilfsmittel Wasserstoffperoxid einsetzt und verwendet.

Die bei der erfindungsgemäßen Verfahrensweise anfallende Abfallschwefelsäure ist durch dieses Oxidationsmittel nicht zusätzlich mit anorganischen Salzen belastet, so daß die Abfallschwefelsäure leicht aufgearbeitet werden kann.

Die erfindungsgemäße Verwendung von Wasserstoffperoxid hat außerdem den Vorteil, daß auch ein Oleum eingesetzt werden kann, das einen nur geringen Schwefeltrioxidgehalt, wie beispielsweise 5 zu 25 Gew.-%, besitzt und daß zudem die Reaktionstemperaturen niedrig, wie insbesondere unter 25° C, gehalten werden können, wobei nachteilige Nebenreaktionen nicht ablaufen und das resultierende Endprodukt eine hohe Reinheit und Qualität aufweist.

Das Wasserstoffperoxid wird dem Oleum als wäßrige Wasserstoffperoxidlösung zugesetzt; hierbei ist darauf zu achten, daß bei der Verdünnung des Oleums mit der wäßrigen Peroxidlösung dieses nicht zu einer Schwefelsäure führt, die weniger als 95 gew.-%ig, insbesondere nicht weniger als 98 gew.-%ig, ist. Bevorzugt sollte das wasserstoffperoxidhaltige Oleum noch mindestens 5%ig an Schwefeltrioxid sein.

In der Regel geht man hierbei so vor, daß man zunächst die Lösung des Di-(arylamino)-benzochinons in Oleum bereitet und zu dieser erst die wäßrige Wasserstoffperoxidlösung zugibt. Im allgemeinen verwendet man pro Mol 2,5-Di-(arylamino)-benzochinon 2 bis 4 Mol, bevorzugt 2,1 bis 3 Mol, Wasserstoffperoxid. Das im erfindungsgemäßen Verfahren eingesetzte Oleum enthält in der Regel 10 bis 50 Gew.-%, bevorzugt 10 bis 30 Gew.-%, Schwefeltrioxid. Es ist vorteilhaft, daß nach dem Zusatz der Peroxidlösung der Schwefeltrioxidgehalt des Reaktionsmediums 5 Gew.-% nicht unterschreitet; jedoch kann die Cyclisierungsreaktion bei der Verwendung von Wasserstoffperoxid als oxidatives Cyclisierungshilfsmittel - abhängig von der eingesetzten Di-(arylamino)-benzochinon-Verbindung - auch bei niedriger-konzentriertem Oleum oder bei bis zu 95 %iger Schwefelsäure durchgeführt werden.

Die Cyclisierungstemperatur wird in der Regel zwischen 0 und 40°C gewählt; vorzugsweise führt man die Cyclisierung zwischen 10 und 30°C, insbesondere zwischen 10 und 25°C, durch. Das Wasserstoffperoxid wird in der handelsüblichen und technisch leicht handhabbaren wäßrigen Lösung mit einem Gehalt von 15 bis 70 Gew.-% Wasserstoffperoxid, bevorzugt mit einem Gehalt von 20 bis 50 Gew.-% Wasserstoffperoxid, eingesetzt.

Als 2,5-Di-(arylamino)-1,4-benzochinon-Verbindungen bzw. deren Kondensationsprodukten aus halogensubstituierten 1,4-Benzochinonen und Arylaminoverbindungen (wobei die Arylreste, wie oben erwähnt, noch substituiert sein können) sind alle diejenigen in das erfindungsgemäße Verfahren einsetzbar, die nicht nur in der oben erwähnten GB-PS 1 589 915, EP-0 141 359A und DE-3 510 613A, sondern auch in den europäischen Patentanmeldungs-Veröffentlichungen Nrs. 0 141 996A, 0 168 751A, 0 212 635A und 0 222 098A beispielhaft oder formelmäßig durch allgemeine Formeln beschrieben sind, desweiteren solche, die der allgemeinen Formel (1)

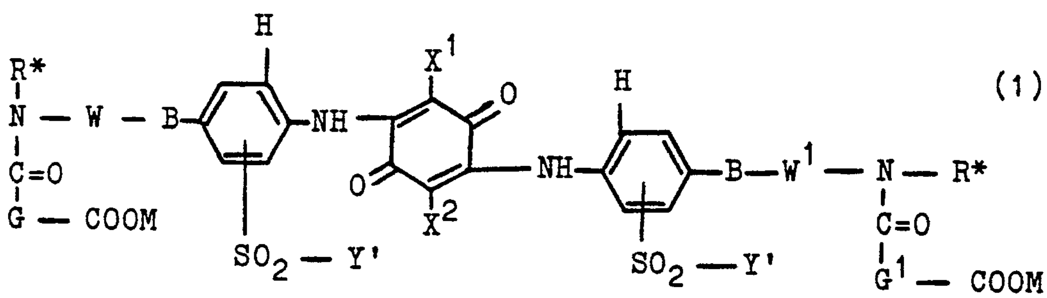

(1)

entsprechen, worin

B eine Aminogruppe der Formel -NH- oder -N(R′)- ist,

in welcher

R′ eine Alkylgruppe von 1 bis 6 C-Atomen, bevorzugt von 1 bis 4 C-Atomen, wie die Methyl- oder Ethylgruppe, ist,

R* ein Wasserstoffatom oder eine gegebenenfalls substituierte Alkylgruppe von 1 bis 4 C-Atomen oder ein gegebenenfalls substituierter Arylrest, wie beispielsweise Methyl, Ethyl, Benzyl, Phenethyl, β-Sulfatoethyl, β-Sulfoethyl, Phenyl, Sulfophenyl oder Sulfobenzyl, ist,

W ein bivalenter, gegebenenfalls substituierter aliphatischer oder gegebenenfalls substituierter araliphatischer oder gegebenenfalls substituierter aromatisch-carbocyclischer oder gegebenenfalls durch Alkyl von 1 bis 4 C-Atomen substituierter ($C_5$-$C_{10}$)-cycloaliphatischer oder gegebenenfalls durch Alkyl von 1 bis 4 C-Atomen substituierter aliphatisch-($C_5$-$C_8$)-cycloaliphatischer Rest ist, wobei die aliphatischen Reste durch Heterogruppen, bevorzugt 1 oder 2 Heterogruppen, unterbrochen sein können, die aus den Gruppen -O- , -S- , $-SO_2-$ , -CO- , -NH-CO- , -CO-NH- , 1,4-Piperidino, -NH- und $-N(R^o)-$ , worin $R^o$ eine der Bedeutungen von R′ hat oder eine Alkanoylgruppe von 2 bis 5 C-Atomen, wie Acetylgruppe, ist, ausgewählt sind, und

$W^1$ eine der für W angegebenen Bedeutungen hat und mit W gleich oder von W verschieden ist, oder die Gruppierung $-B-W^1-N(R^*)-$ und die Gruppierung $-N(R^*)-W-B-$ , zueinander gleich oder voneinander verschieden, jede gemeinsam den bivalenten Rest eines fünf- oder sechsgliedrigen, zwei Stickstoffatome enthaltenden gesättigten Heterocyclus darstellt oder

die Gruppierung $-B-W^1-$ und die Gruppierung -W-B- , zueinander gleich oder voneinander verschieden, jede gemeinsam den bivalenten Rest eines fünf- oder sechsgliedrigen, zwei Stickstoffatome enthaltenden gesättigten Heterocyclus darstellt, der mit einem der beiden Stickstoffatome über eine Alkylengruppe von 2 bis 4 C-Atomen mit der Gruppierung $-N(R^*)-CO-G^1-$ bzw. $-G-CO-A-N(R^*)-$ verbunden ist,

G eine direkte Bindung oder eine geradkettige oder verzweigte Alkylengruppe von 1 bis 8 C-Atomen,

bevorzugt von 2 bis 6 C-Atomen, oder ein aliphatisch-cycloaliphatischer Rest oder ein cycloaliphatischer Rest mit jeweils 5 bis 8 C-Atomen, bevorzugt 6 C-Atomen, im Cycloaliphaten ist,
$G^1$ eine der für G angegebenen Bedeutungen hat und mit G gleich oder von G verschieden ist,
M ein Wasserstoffatom oder ein Alkalimetall, wie Natrium, Kalium und Lithium, oder das Äquivalent eines Erdalkalimetalls, wie beispielsweise des Calciums, insbesondere jedoch ein Alkalimetall ist,
$X^1$ ein Wasserstoffatom oder ein Halogenatom, wie Chlor- oder Bromatom, eine Alkylgruppe von 1 bis 4 C-Atomen, eine Alkoxygruppe von 1 bis 4 C-Atomen, eine Aryloxygruppe oder ein gegebenenfalls substituierter Arylrest, bevorzugt ein Bromatom und insbesondere ein Chloratom, ist,
$X^2$ mit $X^1$ gleich oder von $X^1$ verschieden ist une eine der für $X^1$ angegebenen Bedeutungen hat,
$Y'$ die $\beta$-Hydroxyethyl-Gruppe oder die $\beta$-Sulfatoethyl-Gruppe oder eine Ethylgruppe ist, die in $\beta$-Stellung einen anderen alkalisch eliminierbaren Substituierten besitzt,
die Gruppe $-SO_2-Y'$ bevorzugt in ortho-Stellung zum Rest B gebunden steht
und die einzelnen, auch zweifach erscheinenden Formelglieder zueinander gleiche oder voneinander verschiedene Bedeutungen besitzen können,
wobei die Di-(arylamino)-benzochinon-Verbindungen der allgemeinen Formel (1) zu Triphendioxazinverbindungen entsprechend der allgemeinen Formel (2)

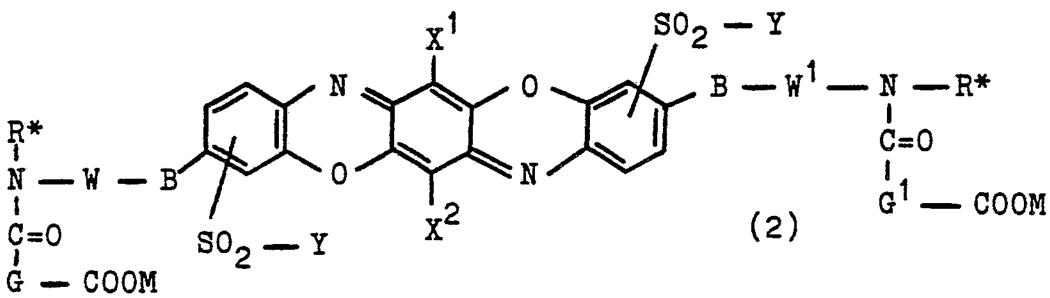
(2)

cyclisieren, in welcher B, G, $G^1$, $R^*$, W, $W^1$, $X^1$ und $X^2$ die obengenannten Bedeutungen haben und Y eine $\beta$-Sulfatoethyl-Gruppe oder eine Ethylgruppe ist, die in $\beta$-Stellung einen anderen alkalisch abspaltbaren Substituenten enthält.
Von den erfindungsgemäß herstellbaren Verbindungen der allgemeinen Formel (2) sind diejenigen der allgemeinen Formel (2a)

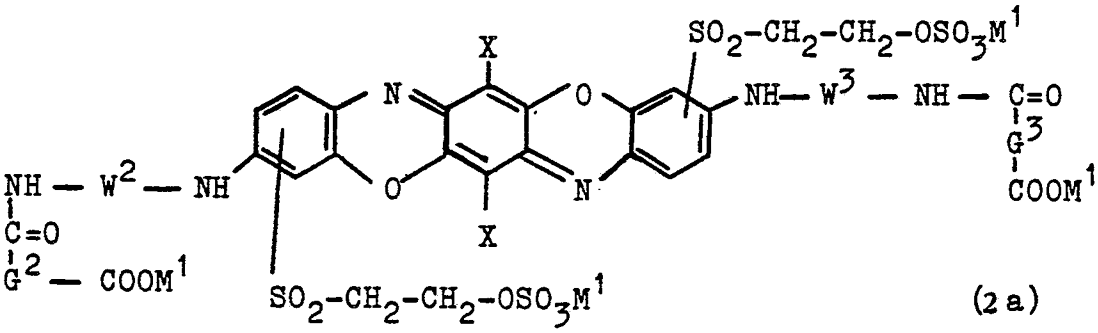
(2 a)

bevorzugt, in welcher
$W^2$ und $W^3$ beide eine Alkylengruppe von 2 bis 6 C-Atomen sind,
$G^2$ und $G^3$ beide eine Alkylengruppe von 2 bis 6 C-Atomen sind,
$M^1$ ein Wasserstoffatom oder bevorzugt ein Alkalimetall ist,
X ein Bromatom oder bevorzugt ein Chloratom ist.

Generell lassen sich nach dem erfindungsgemäßen Verfahren insbesondere solche Triphendioxazinverbindungen herstellen, die der allgemeinen Formel (3)

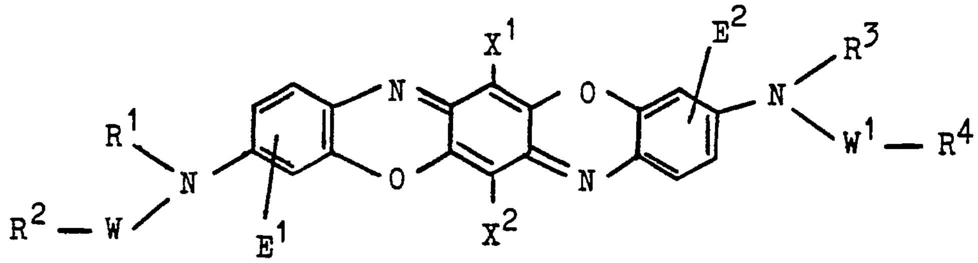

(3)

entsprechen. Als Ausgangsverbindungen hierfür dienen die entsprechenden 2,5-Di-(arylamino)-1,4-benzochinon-Verbindungen der allgemeinen Formel (4)

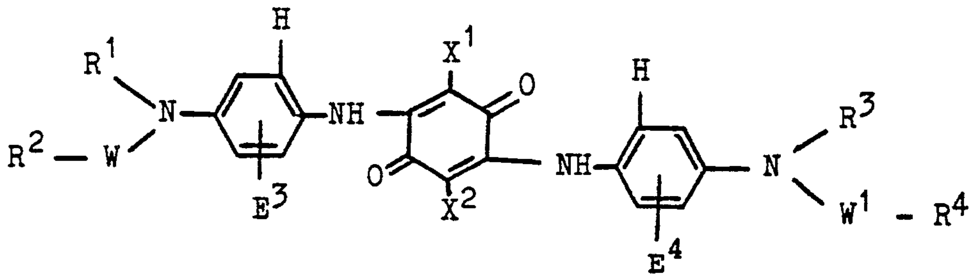

(4)

In den allgemeinen Formeln (3) und (4) bedeuten:

$X^1$ und $X^2$ sowie W und $W^1$ haben eine der für Formel (1) bzw. (2) angegebenen Bedeutungen;

$R^1$ ist ein Wasserstoffatom oder eine gegebenenfalls substituierte Alkylgruppe von 1 bis 4 C-Atomen;

$R^2$ ist eine gegebenenfalls substituierte Alkylgruppe von 1 bis 4 C-Atomen oder eine gegebenenfalls substituierte Arylgruppe;

$R^3$ ist ein Wasserstoffatom oder eine gegebenenfalls substituierte Alkylgruppe von 1 bis 4 C-Atomen;

$R^4$ ist eine gegebenenfalls substituierte Alkylgruppe von 1 bis 4 C-Atomen oder eine gegebenenfalls substituierte Arylgruppe;

$E^1$ ist ein Wasserstoffatom oder eine Carboxygruppe oder bevorzugt eine Sulfogruppe oder eine Gruppe der allgemeinen Formel (5a), (5b), (5c) oder (5d)

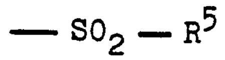 (5a)

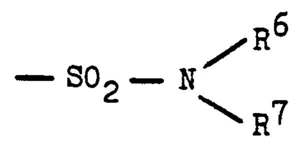 (5b)

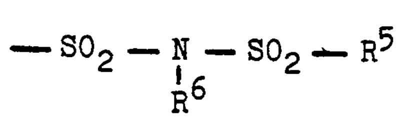 (5c)

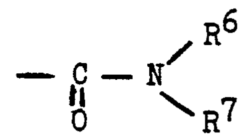 (5d)

in welchen

$R^5$ eine gegebenenfalls substituierte Alkylgruppe von 1 bis 6 C-Atomen oder eine Alkenylgruppe von 2 bis 4 C-Atomen, wie die Vinyl- oder Acrylgruppe, oder eine gegebenenfalls substituierte Arylgruppe,

$R^6$ ein Wasserstoffatom oder eine gegebenenfalls substituierte Alkylgruppe von 1 bis 6 C-Atomen oder eine gegebenenfalls substituierte Arylgruppe, und

$R^7$ ein Wasserstoffatom oder eine gegebenenfalls substituierte Alkylgruppe von 1 bis 6 C-Atomen oder eine gegebenenfalls substituierte Arylgruppe ist;

$R^1$, $R^2$, $R^3$ und $R^4$ bzw. $R^5$, $R^6$ und $R^7$ können zueinander gleiche oder voneinander verschiedene Bedeutungen besitzen;

$E^2$ hat eine der für $E^1$ angegebenen Bedeutungen und ist mit $E^1$ gleich oder von $E^1$ verschieden, vorzugsweise mit $E^1$ gleich;

$E^3$ hat eine der für $E^1$ angegebenen Bedeutungen, wobei die substituierten Alkylgruppen auch Hydroxyalkylgruppen sein können;

$E^4$ hat eine der für $E^3$ angegebenen Bedeutungen und ist mit $E^3$ gleich oder von $E^3$ verschieden, bevorzugt mit $E^3$ gleich.

Gleichzeitig mit der Cyclisierung können gegebenenfalls vorhandene Hydroxyalkylgruppen, wie beispielsweise eine $\beta$-Hydroxyethylgruppe, in die entsprechenden Sulfatoalkylgruppen überführt werden. Bei Cyclisierungstemperaturen von oberhalb 15 °C, insbesondere oberhalb 25 °C, lassen sich auch Sulfogruppen in die gegebenenfalls aromatischen Reste von $E^3$ und $E^4$ sowie $X^1$, $X^2$, $R^2$ und $R^4$ einführen.

In den obigen Formeln (1), (2), (3) und (4) haben die Ausdrücke "substituierte Alkylgruppe von ...", "gegebenenfalls substituierter aliphatischer Rest", "gegebenenfalls substituierte Arylgruppe" etc. insbesondere folgende Bedeutung:

Arylreste in den obengenannten oder nachstehend genannten Gruppen sind insbesondere die Phenyl- und Naphthylreste; sie können substituiert sein, wie beispielsweise durch Substituenten aus der Gruppe Alkyl von 1 bis 4 C-Atomen, wie Methyl und Ethyl, Alkoxy von 1 bis 4 C-Atomen, wie Methoxy und Ethoxy, Halogen, wie Chlor und Brom, Sulfo, Carboxy, einer Gruppe $-SO_2-Y$ mit Y der obengenannten Bedeutung, Sulfamoyl, Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono-oder disubstituiertes Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Sulfamoyl, Trifluormethyl, Benzoylamino, Sulfobenzoylamino, Alkanoylamino von 2 bis 5 C-Atomen, Nitro, Amino und gegebenenfalls mono- oder disubstituiertes Amino, wobei dessen Substituenten gegebenenfalls substituierte aliphatische (einschließlich araliphatische), gegebenenfalls substituierte Aryl- und $(C_5-C_8)$-Cycloalkyl-Reste sind.

Aromatisch-carbocyclische Reste sind beispielsweise Phenylen- und Naphthylen- bzw. Phenyl- und Naphthylreste, die substituiert sein können, wie beispielsweise durch Susbstituenten aus der Gruppe Alkyl von 1 bis 4 C-Atomen, wie Methyl und Ethyl, Alkoxy von 1 bis 4 C-Atomen, wie Methoxy und Ethoxy, Halogen, wie Chlor und Brom, Sulfo, Carboxy, Sulfamoyl, Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Sulfamoyl, Trifluormethyl, Benzoylamino, Sulfobenzoylamino, Alkanoylamino von 2 bis 5 C-Atomen, Nitro, Amino und gegebenenfalls mono- oder disubstituiertes Amino, wobei dessen Substituenten gegebenenfalls substituierte aliphatische (einschließlich araliphatische), gegebenenfalls substituierte Aryl- und $(C_5-C_8)$-Cycloalkyl-Reste sind. Hiervon bevorzugt sind insbesondere solche Phenylen- oder Phenylreste, die durch Substituenten aus der Gruppe Alkyl von 1 bis 4 C-Atomen, Alkoxy von 1 bis 4 C-Atomen und sulfo und/oder durch eine gegebenenfalls mono- oder disubstituierte Aminogruppe substituiert sein können, bspw. durch Alkyl von 1 bis 4 C-Atomen, Phenyl und Benzyl.

Aliphatische Reste sind beispielsweise Alkylgruppen oder Alkylengruppen von jeweils 1 bis 6 C-Atomen, bevorzugt 1 bis 4 C-Atomen, die substituiert sein können. Substituierte Alkyl- und Alkylengruppen sind beispielsweise solche, die durch 1 oder 2 Substituenten aus der Gruppe Chlor, Alkoxy von 1 bis 4 C-Atomen, Benzoylamino, Sulfobenzoylamino, Alkanoylamino von 2 bis 5 C-Atomen, Hydroxy, Sulfato, Phosphato, Phosphono, Acetyloxy, Sulfo, Carboxy oder gegebenenfalls substituiertes Aryl oder durch eine Gruppe $-SO_2-Y$ mit Y der obigen Bedeutung oder durch eine Gruppe der oben genannten und definierten allgemeinen Formel (5d) substituiert sein können. Bevorzugte Substituenten sind hiervon die Carboxy- und Sulfogruppen sowie Sulfatogruppen.

Die Formelglieder W und $W^1$ sind beispielsweise Alkylengruppen von 1 bis 6 C-Atomen, insbesondere von 2 bis 4 C-Atomen, wobei die Alkylenkette durch 1 oder 2 Heterogruppen, die bevorzugt aus den Gruppen -O- , -NH- , $-SO_2-$ , -NH-CO- , -CO-NH- und $-N(R')-$ mit $R'$ der obigen Bedeutung ausgewählt sind, unterbrochen sein kann, oder ein Alkylen-phenylen-, ein Phenylen-alkylen-, ein Phenylen-alkylen-phenylen- oder Alkylen-phenylen-alkylen-Rest, wobei in diesen araliphatischen Resten die Alkylenreste solche von 1 bis 6, bevorzugt 1 bis 4 C-Atomen sind und gegebenenfalls durch die angegebenen Substituenten substituiert und/oder durch eine oder zwei der genannten Heterogruppen unterbrochen sein können und die Benzolkerne jeweils noch durch 1 oder 2 Substituenten substituiert sein können, die aus der Gruppe der Substituenten von Sulfo, Carboxy, Sulfamoyl, Carbamoyl, Methyl, Ethyl, Methoxy, Ethoxy, Nitro, Chlor, Amino und durch gegebenenfalls substituierte aliphatische und/oder gegebenenfalls substituierte Arylreste substituiertes Amino ausgewählt sind, wobei im Falle, daß eine Alkylengruppe durch Heterogruppen unterbrochen ist, deren Alkylenanteile darin bevorzugt solche von 2 oder 3 C-Atomen sind, und wobei die aliphatischen und Arylreste noch durch ein Sauerstoffatom oder eine Gruppe -NH- verbunden sein können. Die Formelglieder W und $W^1$ sind weiterhin beispielsweise ein Phenylenrest, insbesondere ein meta- oder para-Phenylenrest, der noch durch 1 oder 2 Substituenten aus der Gruppe Sulfo, Carboxy, Sulfamoyl, Carbamoyl, Methyl, Ethyl, Methoxy, Ethoxy, Chlor, Amino und durch gegebenenfalls substituierte aliphatische und/oder gegebenenfalls substituierte Arylreste substituiertes Amino, bevorzugt jedoch durch Sulfo, substituiert sein kann, oder ein gegebenenfalls durch Sulfo substituierter Naphthylenrest. Bevorzugt ist W bzw. $W^1$ ein Alkylenrest von 2 bis 4 C-Atomen, der durch 1 oder 2 Substituenten, bevorzugt einen Substituenten, aus der Gruppe Sulfo, Sulfato, Carboxy, Phenyl und Sulfophenyl substituiert sein kann, wobei die Alkylenreste auch durch ein Sauerstoffatom oder eine Gruppe -NH- verbunden sein können, oder ein Alkylenphenylen-Rest mit solchen Alkylenresten.

Reste $W^1$ sind beispielsweise der meta- oder para-Phenylenrest, der 1,2-Ethylen-, 1,3-Propylen-, 1,4-Butylen-, 1,5-Pentylen-, 1,6-Hexylen-, 2-Methyl-1,3-propylen-, 2-Sulfophenyl-1,3-propylen- und 2-Sulfato-1,3-propylen-Rest, desweiteren beispielsweise ein sulfosubstituierter 1,4-Phenylenrest, ein 1,4- und 1,3-Cyclohexylenrest, ein bivalenter Rest der nachstehend angegebenen Formeln (a) bis (z), hiervon bevorzugt der 1,2-Ethylen-, 1,3-Propylen- und 1,4-Butylen-Rest, ein Rest der Formeln (a), ein 2-Sulfophenyl-1,3-propylen- und ein 2-Sulfato-1,3-propylen-Rest und ein sulfosubstituierter 1,4-Phenylenrest:

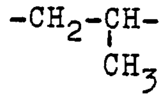
$(a_1)$

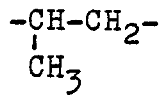
$(a_2)$

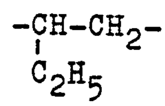
$(b_1)$

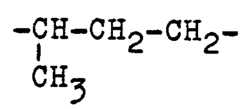
$(b_2)$

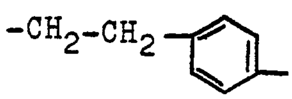
(c)

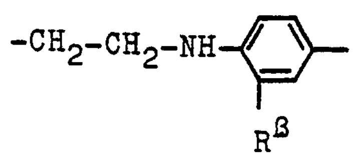
$(d_1)$

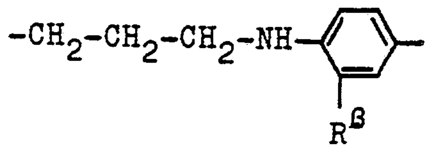

($d_2$)

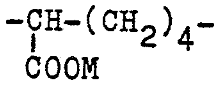

(e)

$-CH_2-CH_2-O-CH_2-CH_2-$ (f)

$-CH_2-CH_2-S-CH_2-CH_2-$ ($g_1$)

$-CH_2-CH_2-SO_2-CH_2-CH_2-$ ($g_2$)

$-(CH_2)_3-O-CH_2-CH_2-O-(CH_2)_3-$ (h)

$-CH_2-CH_2-NH-CH_2-CH_2-$ (j)

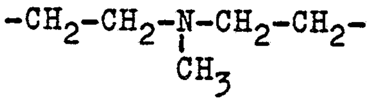

(k)

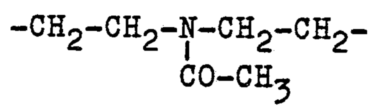

(m)

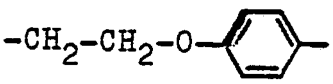

(n)

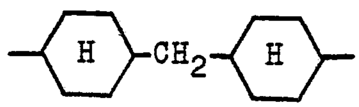

(p)

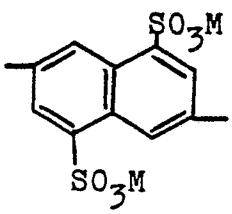

(q)

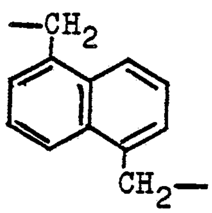

(r)

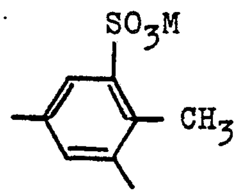

(s)

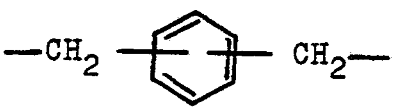

(t)

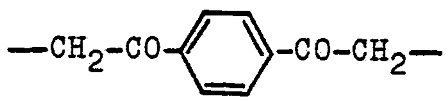

(u)

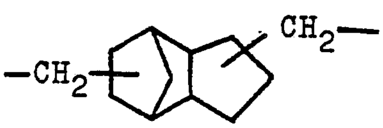

(v)

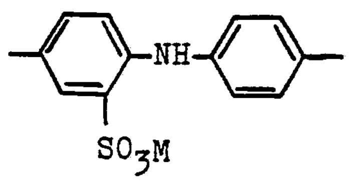

(w)

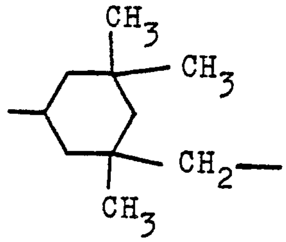
(x)

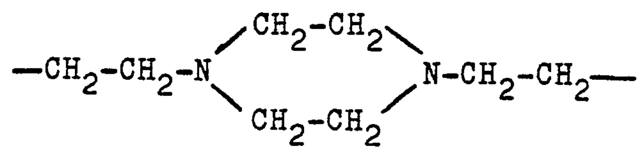
(y)

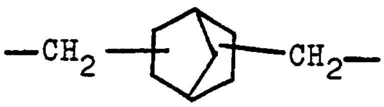
(z)

in welchen $R^{\beta}$ ein Wasserstoffatom oder eine Sulfogruppe ist und M ein Wasserstoffatom oder ein Alkalimetall, wie Natrium, Kalium und Lithium, oder das Äquivalent eines Erdalkalimetalls, wie beispielsweise des Calciums, insbesondere jedoch ein Alkalimetall ist.

Reste W sind beispielsweise die eben für $W^1$ genannten, jedoch "spiegelbildlich" angeordneten Gruppen.

Sofern die Formelreste E substituierte Sulfonamidgruppen sind, ist deren N-Atom durch gegebenenfalls substituierte Alkylgruppen von 1 bis 6 C-Atomen (einschließlich gegebenenfalls substituiertes Aralkyl) und gegebenenfalls substituierte Arylreste mono- oder disubstituiert, wobei die Substituenten bevorzugt wasserlöslichmachende Gruppen, wie die Sulfo-, Carboxy-, Sulfato-, Phosphato oder Phosphono-Gruppe sind. Bevorzugt ist die gegebenenfalls substituierte Sulfonamidgruppe eine Gruppe der allgemeinen Formel $-SO_2-NR^6R^7$ , in welcher $R^6$ ein Wasserstoffatom oder eine Alkylengruppe von 2 bis 6 C-Atomen, bevorzugt von 2 bis 4 C-Atomen, ist, die durch eine Sulfo- oder Sulfatogruppe substituiert ist, wobei die Alkylengruppe noch durch 1 oder 2 Heterogruppen, die aus den Gruppen der Formeln -O- , -S- , -NH- und $-N(R')-$ mit $R'$ der oben angegebenen Bedeutung ausgewählt sind, unterbrochen sein kann, oder ist ein Alkylenrest von 2 bis 6 C-Atomen, bevorzugt 2 bis 4 C-Atomen, der durch eine Carboxy- oder Phosphatogruppe substituiert ist, oder ist ein Alkylenrest von 1 bis 3 C-Atomen, der durch eine Phosphonogruppe substituiert ist, oder ist ein Naphthyl- oder Phenylrest, die beide durch Substituenten aus der Gruppe Methyl, Methoxy, Ethoxy, Chlor, Carboxy und Sulfo substituiert sein können, wobei die Substituenten bevorzugt 1 bis 3 Sulfogruppen sind und hiervon der Monosulfo- und Disulfophenyl-Rest bevorzugt ist, und worin $R^7$ für ein Wasserstoffatom steht oder eine Alkylgruppe von 1 bis 6 C-Atomen, bevorzugt 2 bis 4 C-Atomen, bedeutet, die substituiert sein kann, bevorzugt durch 1 oder 2, insbesondere durch 1 Substituenten aus der Gruppe Hydroxy, Sulfato, Carboxy, Sulfo und Methoxy. Substituierte Sulfonamidgruppen der allgemeinen Formel $-SO_2-NH-SO_2-R^5$ sind beispielsweise solche, in welcher $R^5$ eine gegebenenfalls substituierte Alkylgruppe von 1 bis 6 C-Atomen, bevorzugt 1 bis 4 C-Atomen, insbesondere Methylgruppe, bedeutet oder einen gegebenenfalls substituierten Arylrest darstellt, wobei der Arylrest insbesondere bevorzugt ein Phenylrest ist, der durch 1 oder 2 Sulfogruppen substituiert sein kann.

Substituierte Alkylsulfonylgruppen, wie beispielsweise die der allgemeinen Formel (5a), sind insbesondere auch die Methylsulfonyl-, β-Sulfoethylsulfonyl-, β-Carboxyethylsulfonyl-, β-Sulfatoethylsulfonyl- und β-Phosphatoethylsulfonyl-Gruppe.

Von den Gruppen der Formeln (5b) und (5d) können insbesondere die (β-Sulfatoethylsulfonyl)-phenylamido-sulfonyl- bzw. (β-Sulfatoethylsulfonyl)-phenylamido-carbonyl-Gruppe hervorgehoben werden.

Gruppen entsprechend den Formelresten $R^2$-W- und $-W^1-R^4$ sind insbesondere β-Aminoethyl-, β-Sulfatoethyl-, γ-Amino-propyl-, γ-Hydroxy-propyl-, β-Carboxy-ethyl-, γ-(β'-Carboxy-propionylamino)-propyl und β-(β'-Carboxy-propionylamino)-ethyl-Gruppen.

Bevorzugt ist W bzw. $W^1$ in den allgemeinen Formeln (1) und (2) ein Alkylenrest von 2 bis 4 C-Atomen oder, falls er durch eine oder zwei Heterogruppen unterbrochen ist, ein Alkylenrest von 2 bis 6, insbesondere von 2 bis 4 C-Atomen, wobei im Alkylenrest die Heterogruppe bevorzugt ein Sauerstoffatom oder die Gruppe -NH- oder $-N(CH_3)-$ ist. Weiterhin sind W bzw. $W^1$ beispielsweise Cycloalkylengruppen von 5 oder 6 C-Atomen mit 1 bis 3 Methylgruppen als Substituenten oder zwei solche mit einem Alkylenrest von 1 bis 4 C-Atomen verbundene Cycloalkylengruppen oder ein Alkylenrest von 2 bis 6 C-Atomen, der durch eine solche Cycloalkylengruppe unterbrochen sein kann.

Bevorzugte Reste $W^1$ in den Formeln (1) und (2) sind beispielsweise der 1,2-Ethylen-, 1,3-Propylen-, 1,4-Butylen-, 1,5-Pentylen-, 1,6-Hexylen-, 2-Methyl-1,3-propylen-, 1,2-Dimethyl-1,2-ethylen-, 1,3-Dimethyl-1,3-propylen-, 2,2-Dimethyl-1,3-propylen-oder 1,2-, 1,4- und 1,3-Cyclohexylen-Rest oder ein bivalenter Rest der nachstehend angegebenen Formeln (a′) bis (s′), hiervon bevorzugt der 1,2-Ethylen-, 1,3-Propylen- und 1,4-Butylen-Rest und der Rest der Formel (a′):

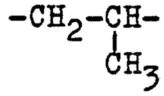
(a′)

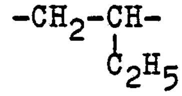
(b′)

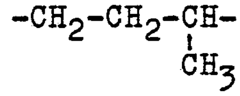
(c′)

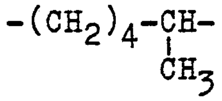
(d′)

$-CH_2-CH_2-O-CH_2-CH_2-$ (e′)

$-CH_2-CH_2-S-CH_2-CH_2-$ (f′)

$-CH_2-CH_2-SO_2-CH_2-CH_2-$ (g′)

$-(CH_2)_3-O-CH_2-CH_2-O-(CH_2)_3-$ (h′)

$-CH_2-CH_2-NH-CH_2-CH_2-$ (j′)

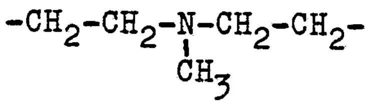
(k′)

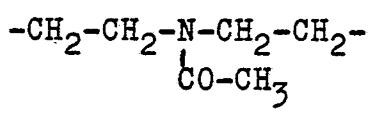
(m′)

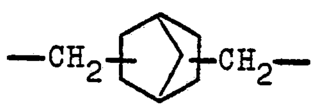
(n′)

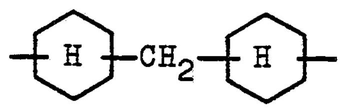
(p′)

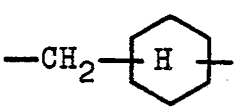
(r′)

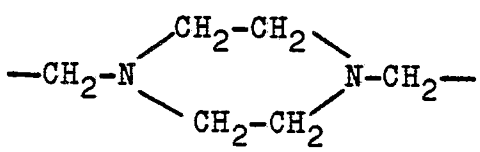
(s′).

Die Reste W sind beispielsweise die eben für W[1] genannten, jedoch "spiegelbildlich" angeordneten Gruppen.

Heterocyclische Gruppierungen, die sich gemeinsam aus den Formelresten -B-W[1]-N(R*)- bzw. -N(R*)-W-B- bilden, sind beispielsweise der bivalente 1,4-Piperazino-Rest. Gruppierungen mit heterocyclischen Resten, die sich gemeinsam aus dem Formelrest -B-W[1]- bilden, sind beispielsweise der Rest der Formel (t′)

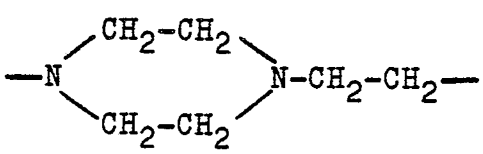
.
(t′)

Gruppierungen mit heterocyclischen Resten, die sich gemeinsam aus dem Formelrest -W-B- bilden, sind beispielsweise ein Rest der Formel (u′)

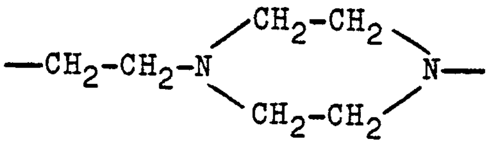

(u').

Aliphatische und cycloaliphatische Reste $G^1$ sind beispielsweise der 1,2-Ethylen-, 1,3-Propylen-, 1,4-Butylen-, 1,5-Pentylen-, 2-Methyl-1,3-propylen-, 1,2-Dimethyl-1,2-ethylen-, 2-Ethyl-1,3-propylen-, 2,2-Dimethyl-1,3-propylen-, 2-(n- oder iso-Propyl)-1,3-propylen-, 1,2,2,-Trimethyl-1,2-ethylen-, 1,2-Diethyl-1,2-ethylen-, 1,1,2,2-Tetramethyl-1,2-ethylen-, 2-Methyl-2-ethyl-1,3-propylen-, 1,1,3,3-Tetra-methyl-1,3-propylen-, 1,2-Cyclohexylen- oder 1,4-Cyclohexylen-Rest oder ein bivalenter Rest der nachstehend angegebenen Formeln (a*) bis (h*), hiervon bevorzugt der 1,2-Ethylen-, 1,3-Propylen-, 2-Methyl-1,3-propylen-, 2,2-Dimethyl-1,3-propylen- und 1,2-Cyclohexylenrest und der Rest der Formel (a*):

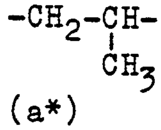

(a*)

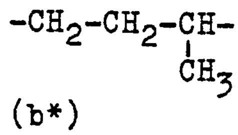

(b*)

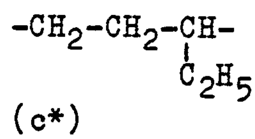

(c*)

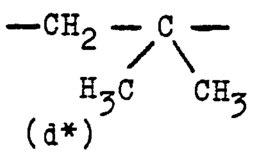

(d*)

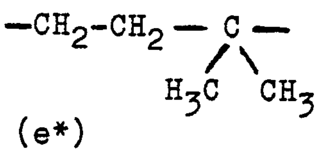

(e*)

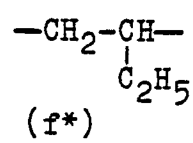

(f*)

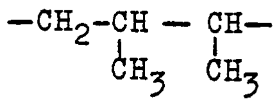

(g*)

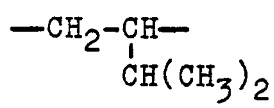

(h*)

Die Reste G sind beispielsweise die eben für $G^1$ genannten, jedoch "spiegelbildlich" angeordneten Gruppen.

Die nach dem erfindungsgemäßen Verfahren erhältlichen Triphendioxazinverbindungen können, analog den Angaben aus dem Stand der Technik, zum Färben von hydroxy- und/oder carbonamidgruppenhaltigen Materialien, insbesondere Fasermaterialien, wie Wolle, Baumwolle und synthetische Polyamidfasern, verwendet werden oder sie können, abhängig davon, ob sie eine entsprechende funktionelle Gruppe besitzen, gemäß bekannten Verfahrensweisen in Farbstoffe mit einer anderen Gruppe übergeführt werden, so beispielsweise im Falle des Vorhandenseins von Aminogruppen durch Umsetzung von halogenhaltigen Heterocyclen, wie Trihalogentriazinen, oder von anderen faserreaktiven Acylierungsmitteln, wie aromatischen, eine faserreaktive Gruppe enthaltenden Carbonsäurechloriden, in entsprechende Reaktivfarbstoffe mit einer bekannten Reaktivgruppe übergeführt werden.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile stehen zu Volumenteilen im Verhältnis von Kilogramm zu Liter.

Beispiel 1

a) 178 Teile 2,5-Bis-{4-N-[β-(bernsteinsäure-monoamido)-ethyl]-amino-3-(β-hydroxyethylsulfonyl)-phenylamino}-3,6-dichlor-1,4-benzochinon, das wie im nachfolgenden Abschnitt b) beschrieben hergestellt werden kann, werden in 2000 Teile 15 %igem Oleum bei 20 bis 25 °C eingetragen; man rührt bis zur

vollständigen Lösung nach. Sodann läßt man innerhalb 45 Minuten bei 20 bis 25 °C unter Kühlung 39 Teile einer 35 %igen wäßrigen Wasserstoffperoxidlösung zulaufen, rührt den Reaktionsansatz noch eine Stunde nach und gibt ihn sodann auf 3800 Teile Eis, wobei darauf geachtet werden soll, daß die Temperatur 20 °C nicht übersteigt. Die ausgefallene Verbindung wird abfiltriert, in etwa 800 Teilen Wasser aufgenommen und mit Natriumcarbonat durch Einstellen eines pH-Wertes von 4,8 gelöst. Hieraus kann die Triphendioxazinverbindung der Formel

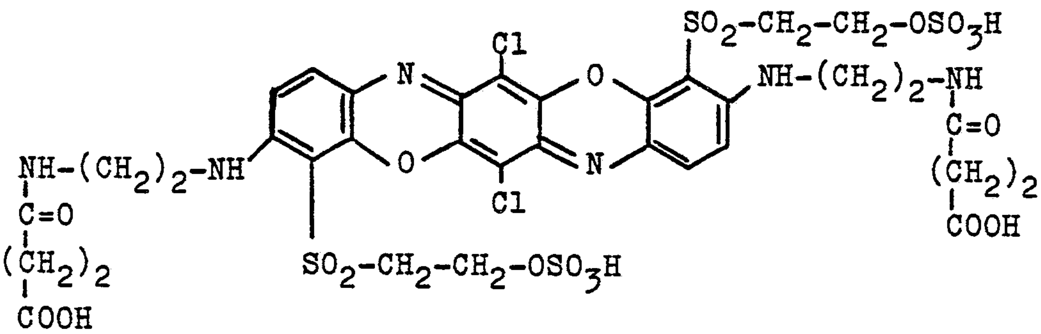

als elektrolytsalzhaltiges (vorwiegend natriumsulfathaltiges) Natriumsalz isoliert werden (in der obigen Formel kann die jeweilige β-Sulfatoethylsulfonyl-Gruppe auch in der anderen ortho-Stellung zur Aminogruppe gebunden sein; sie befindet sich jedoch mit größerer Wahrscheinlichkeit in der in der obigen Formel angegebenen Stellung).

Als Nebenprodukt entsteht eine etwa 35%ige wäßrige Schwefelsäure, die praktisch keine anorganischen Salze enthält und auf üblichem Wege leicht aufgearbeitet werden kann.

b) Die unter a) eingesetzte Bis-phenylamino-benzochinon-Verbindung kann wie folgt, ausgehend von 4-(β-Amino-ethyl-amino)-3-(β-hydroxyethylsulfonyl)-nitro-benzol (herstellbar analog den Angaben der deutschen Offenlegungsschrift Nr. 3 502 991) synthetisiert werden: Zu einer 40 °C warmen Suspension von 60 Teilen dieser Nitrobenzol-Verbindung in 200 Teilen Wasser gibt man langsam eine Lösung von 22 Teilen Bernsteinsäureanhydrid in 40 Teilen Aceton; man rührt den Ansatz bei 40 °C noch 30 Minuten weiter und isoliert nach Abkühlen das ausgefallene Produkt in üblicher Weise. 39 Teile dieser Bernsteinsäure-monoamid-nitrobenzol-Verbindung werden in 200 Volumenteilen Methanol gelöst und in einem Autoklaven bei einer Temperatur von bis zu 80 °C und einem Druck von 50 bar in Gegenwart eines Palladiumkatalysators während 4 Stunden hydriert. Das Produkt wird in üblicher Weise aus dem Hydrieransatz isoliert. Die gewünschte Verbindung der Formel

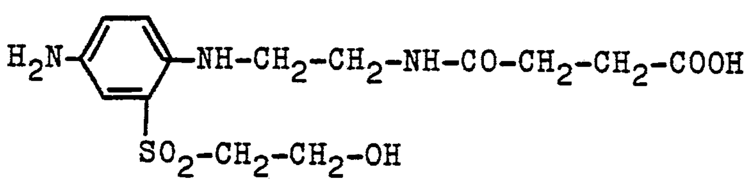

wird in Form eines Öles erhalten.

54 Teile dieser Anilinverbindung werden in 500 Teilen Wasser bei einem pH-Wert von 6,0 gelöst. Man erwärmt diese Lösung auf 60 °C und setzt bei dieser Temperatur unter Einhaltung eines pH-Wertes zwischen 6,0 und 6,5 portionsweise insgesamt 19 Teile Chloranil hinzu. Der Ansatz wird noch 3 Stunden nachgerührt, das ausgefallene Produkt abgesaugt, mit etwas Wasser gewaschen und unter reduziertem Druck bei 80 °C getrocknet.

Die $^{1}H$-NMR-spektroskopische Analyse stimmte mit der Konstitution der unter a) angegebenen Bis-phenylamino-benzochinon-Verbindung überein.

Beispiel 2

9,5 Teile 2,5-Bis-{4-N-[γ-(bernsteinsäure-monoamido)-propyl]-amino-3-(β-hydroxyethylsulfonyl)-phenylamino}-3,6-dichlor-1,4-benzochinon werden in 95 Teilen 20%igem Oleum bei 20 bis 25 °C eingetragen; der Ansatz wird bis zur vollständigen Lösung nachgerührt. Sodann läßt man unter Kühlung bei 20 bis 25 °C 1,3 Teile einer 35%igen wäßrigen Wasserstoffperoxidlösung zulaufen und rührt den Reaktionsansatz noch eine Stunde nach; er wird anschließend auf 200 Teile Eis gegeben, wobei darauf zu achten ist, daß die Temperatur 20 °C nicht übersteigt. Die ausgefallene Verbindung wird abfiltriert, in Wasser aufgenommen und mit Natriumcarbonat bei einem pH-Wert von 4,8 gelöst. Aus dieser Lösung wird die Verbindung der Formel

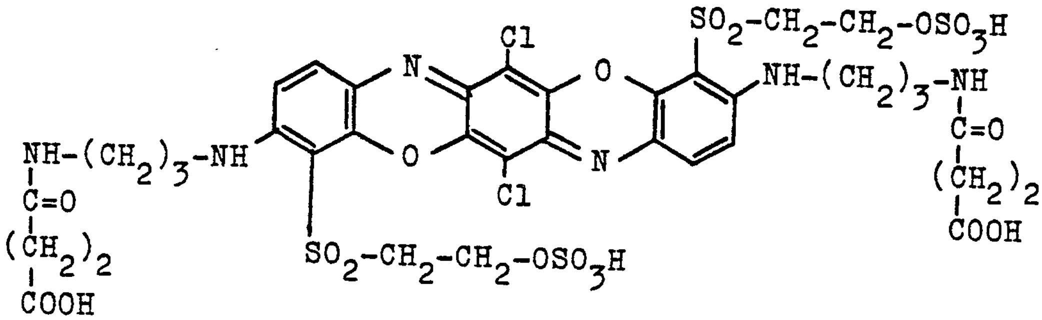

als elektrolytsalzhaltiges Natriumsalz durch Sprühtrocknung isoliert.

Als Nebenprodukt entsteht eine etwa 35 %ige wäßrige Schwefelsäure, die praktisch keine anorganischen Salze enthält und auf üblichem Wege leicht aufgearbeitet werden kann.

Die obengenannte Bis-phenylamino-benzochinon-Ausgangsverbindung kann analog den Angaben des Beispieles 1b), ausgehend von der Verbindung 3-(β-Hydroxyethylsulfonyl)-4-N-[γ-(bernsteinsäure-monoamido)-n-propyl-amino]-nitrobenzol, synthetisiert werden.

Beispiele 3 bis 17

In den nachfolgenden Tabellenbeispielen sind weitere Triphendioxazin-Verbindungen mit Hilfe der Formelglieder der allgemeinen Formel (5)

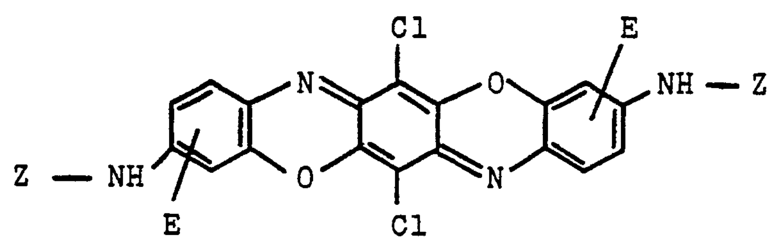

(5)

genannt, die sich in erfindungsgemäßer Weise, wie analog den obigen Ausführungsbeispielen, aus den entsprechenden Ausgangsverbindungen der allgemeinen Formel (6)

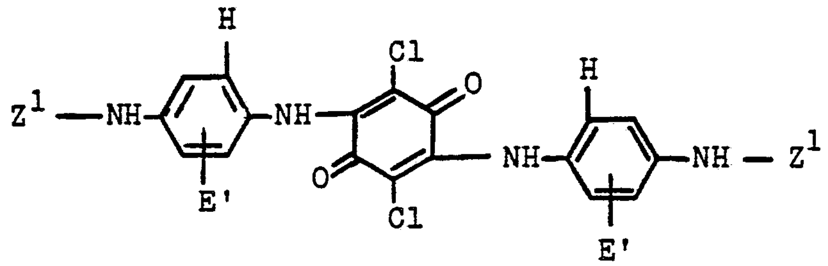

(6)

(in welcher E' die in der jeweiligen Verbindung entsprechende Bedeutung von E besitzt oder, falls E eine Sulfatogruppe enthält, auch das entsprechende Hydroxy-derivat darstellen kann und in welcher Z' die für die jeweilige Verbindung entsprechende Bedeutung von Z besitzt oder, falls diese Gruppe eine Sulfatogruppe enthält, auch das entsprechende Hydroxy-Derivat bedeuten kann) herstellen lassen.

| Bsp. | Gruppe E bzw. E' | Gruppe Z bzw. $Z^1$ |
|---|---|---|
| 3 | ß-Sulfoethylsulfonyl | ß-[4-(ß'-Sulfatoethyl-sulfonyl)-phenyl]-ethyl |
| 4 | dito | 3-(ß-sulfatoethylsulfonyl)-4-N-[ß-(sulfatoethyl)]-amino-phenyl |
| 5 | dito | 3-Sulfo-4-amino-phenyl |
| 6 | dito | ß-Amino-ethyl |
| 7 | dito | γ-Amino-propyl |
| 8 | dito | ß-Amino-propyl |
| 9 | Sulfo | ß-[5-(ß-Sulfatoethylsulfonyl)-benz-1,2,3-triazol-1-yl]-ethyl |
| 10 | Sulfo | ß-[5-(γ-Sulfatoethylsulfonyl)-benz-1,2,3-triazol-1-yl]-propyl |
| 11 | ß-Sulfoethyl | dito |
| 12 | ß-Sulfatoethylsulfonyl | ß-(5-Sulfo-benz-1,2,3-triazol-1-yl)-ethyl |
| 13 | dito | Methyl |
| 14 | dito | Ethyl |
| 15 | dito | 3-Sulfo-4-amino-phenyl |
| 16 | dito | ß-[N-(ß-Sulfatoethyl)]-amino-ethyl |
| 17 | dito | ß-[N-(ß-Sulfatopropyl)]-amino-ethyl |

## Ansprüche

1. Verfahren zur Herstellung von anionischen Triphendioxazinverbindungen durch Cyclisierungsreaktion von 2,5-Di-(arylamino)-1,4-benzochinon-Verbindungen, wobei deren Arylreste weitere Substituenten enthalten können, in Oleum oder 95 bis 100%iger Schwefelsäure als Cyclisierungsmedium, dadurch gekennzeichnet, daß man als oxidierendes Cyclisierungshilfsmittel Wasserstoffperoxid einsetzt und verwendet.

2. Verfahren nach Anspruch 1, dadurch gekennzeichnet, daß die Arylreste der Ausgangsverbindungen als Substituenten Sulfonsäuregruppen und/oder Sulfonylgruppen enthalten.

3. Verfahren nach Anspruch 2, dadurch gekennzeichnet, daß die Sulfonylgruppen gegebenenfalls substituierte Alkylsulfonylgruppen oder gegebenenfalls substituierte Sulfonamidgruppen sind.

4. Verfahren nach Anspruch 2, dadurch gekennzeichnet, daß die Sulfonylgruppen β-Hydroxyethylsulfonyl-Gruppen und/oder faserreaktive Gruppen aus der Vinylsulfonreihe sind.

5. Verfahren nach mindestens einem der Ansprüche 1 bis 4, dadurch gekennzeichnet, daß man die Umsetzung in wasserstoffperoxid-haltiger Schwefelsäure mit einem Gehalt von mindestens 5 % Schwefeltrioxid durchführt.

6. Verfahren nach Anspruch 1 oder 5, dadurch gekennzeichnet, daß die verfahrensgemäß hergestellten anionischen Triphendioxazinverbindungen und die 2,5-Di-(arylamino)-1,4-benzochinon-Ausgangsverbindungen bzw. Kondensationsprodukte aus halogensubstituierten 1,4-Benzochinonen und Arylaminoverbindungen solche entsprechend der britischen Patentschrift 1 589 915 sind.

7. Verfahren nach Anspruch 1 oder 5, dadurch gekennzeichnet, daß die verfahrensgemäß hergestellten anionischen Triphendioxazinverbindungen und die 2,5-Di-(arylamino)-1,4-benzochinon-Ausgangsverbindungen bzw. Kondensationsprodukte aus halogensubstituierten 1,4-Benzochinonen und Arylaminoverbindungen solche entsprechend der europäischen Patentanmeldungs-Veröffentlichung Nr. 0 141 359A sind.

8. Verfahren nach Anspruch 1 oder 5, dadurch gekennzeichnet, daß die verfahrensgemäß hergestellten anionischen Triphendioxazinverbindungen und die 2,5-Di-(arylamino)-1,4-benzochinon-Ausgangsverbindungen bzw. Kondensationsprodukte aus halogensubstituierten 1,4-Benzochinonen und Arylaminoverbindungen solche entsprechend der deutschen Offenlegungsschrift 3 510 613 sind.

9. Verfahren nach Anspruch 1 oder 5, dadurch gekennzeichnet, daß die verfahrensgemäß hergestellten anionischen Triphendioxazinverbindungen und die 2,5-Di-(arylamino)-1,4-benzochinon-Ausgangsverbindungen bzw. Kondensationsprodukte aus halogensubstituierten 1,4-Benzochinonen und Arylaminoverbindungen solche entsprechend der europäischen Patentanmeldungs-Veröffentlichung Nr. 0 141 996A sind.

10. Verfahren nach Anspruch 1 oder 5, dadurch gekennzeichnet, daß die verfahrensgemäß hergestellten anionischen Triphendioxazinverbindungen und die 2,5-Di-(arylamino)-1,4-benzochinon-Ausgangsverbindungen bzw. Kondensationsprodukte aus halogensubstituierten 1,4-Benzochinonen und Arylaminoverbindungen solche entsprechend der europäischen Patentanmeldungs-Veröffentlichung Nr. 0 168 751A sind.

11. Verfahren nach Anspruch 1 oder 5, dadurch gekennzeichnet, daß die verfahrensgemäß hergestellten anionischen Triphendioxazinverbindungen und die 2,5-Di-(arylamino)-1,4-benzochinon-Ausgangsverbindungen bzw. Kondensationsprodukte aus halogensubstituierten 1,4-Benzochinonen und Arylaminoverbindungen solche entsprechend der europäischen Patentanmeldungs-Veröffentlichung Nr. 0 212 635A sind.

12. Verfahren nach Anspruch 1 oder 5, dadurch gekennzeichnet, daß die verfahrensgemäß hergestellten anionischen Triphendioxazinverbindungen und die 2,5-Di-(arylamino)-1,4-benzochinon-Ausgangsverbindungen bzw. Kondensationsprodukte aus halogensubstituierten 1,4-Benzochinonen und Arylaminoverbindungen solche entsprechend der europäischen Patentanmeldungs-Veröffentlichung Nr. 0 222 098A sind.

13. Verfahren nach Anspruch 1 oder 5, dadurch gekennzeichnet, daß man eine Verbindung der allgemeinen Formel (1)

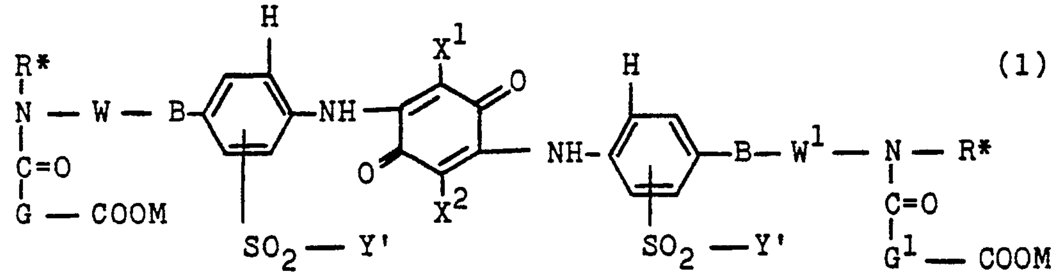

(1)

in welcher
B eine Aminogruppe der Formel -NH- oder $-N(R')-$ ist, in welcher
$R'$ eine Alkylgruppe von 1 bis 6 C-Atomen, bevorzugt von 1 bis 4 C-Atomen, ist,
$R^*$ ein Wasserstoffatom oder eine gegebenenfalls substituierte Alkylgruppe von 1 bis 4 C-Atomen oder ein gegebenenfalls substituierter Arylrest ist,
W ein bivalenter, gegebenenfalls substituierter aliphatischer oder gegebenenfalls substituierter araliphatischer oder gegebenenfalls substituierter aromatisch-carbocyclischer oder gegebenenfalls durch Alkyl von 1 bis 4 C-Atomen substituierter $(C_5-C_{10})$-cycloaliphatischer oder gegebenenfalls durch Alkyl von 1 bis 4 C-Atomen substituierter aliphatisch-$(C_5-C_8)$-cycloaliphatischer Rest ist, wobei die aliphatischen Reste durch Heterogruppen, bevorzugt 1 oder 2 Heterogruppen, unterbrochen sein können, die aus den Gruppen -O- , -S- , $-SO_2-$ , -CO- , -NH-CO- , -CO-NH- , 1,4-Piperidino, -NH- und $-N(R^o)-$ , worin $R^o$ eine der Bedeutungen von $R'$ hat oder eine Alkanoylgruppe von 2 bis 5 C-Atomen ist, ausgewählt sind, und
$W^1$ eine der für W angegebenen Bedeutungen hat und mit W gleich oder von W verschieden ist, oder
die Gruppierung $-B-W^1-N(R^*)-$ und die Gruppierung $-N(R^*)-W-B-$ , zueinander gleich oder voneinander verschieden, jede gemeinsam den bivalenten Rest eines fünf- oder sechsgliedrigen, zwei Stickstoffatome enthaltenden gesättigten Heterocyclus darstellt oder
die Gruppierung $-B-W^1-$ und die Gruppierung -W-B- , zueinander gleich oder voneinander verschieden, jede gemeinsam den bivalenten Reste eines fünf- oder sechsgliedrigen, zwei Stickstoffatome enthaltenden gesättigten Heterocyclus darstellt, der mit einem der beiden Stickstoffatome über eine Alkylengruppe von 2 bis 4 C-Atomen mit der Gruppierung $-N(R^*)-CO-G^1$-bzw. $-G-CO-A-N(R^*)-$ verbunden ist,
G eine direkte Bindung oder eine geradkettige oder verzweigte Alkylengruppe von 1 bis 8 C-Atomen, bevorzugt von 2 bis 6 C-Atomen, oder ein aliphatischcycloaliphatischer Rest oder ein cycloaliphatischer Rest mit jeweils 5 bis 8 C-Atomen, bevorzugt 6 C-Atomen, im Cycloaliphaten ist,
$G^1$ eine der für G angegebenen Bedeutungen hat und mit G gleich oder von G verschieden ist,
M ein Wasserstoffatom oder ein Alkalimetall oder das Äquivalent eines Erdalkalimetalls, bevorzugt ein Alkalimetall,
$X^1$ ein Wasserstoffatom oder ein Halogenatom, eine Alkylgruppe von bis 1 bis 4 C-Atomen, eine Alkoxygruppe von 1 bis 4 C-Atomen, eine Aryloxygruppe oder ein gegebenenfalls substituierter Arylrest, bevorzugt ein Bromatom oder ein Chloratom, ist,
$X^2$ mit $X^1$ gleich oder von $X^1$ verschieden ist und eine der für $X^1$ angegebenen Bedeutungen hat,
$Y'$ die $\beta$-Hydroxyethyl-Gruppe oder die $\beta$-Sulfatoethyl-Gruppe oder eine Ethylgruppe ist, die in $\beta$-Stellung einen anderen alkalisch eliminierbaren Substituenten besitzt,
die Gruppe $-SO_2-Y'$ bevorzugt in ortho-Stellung zum Rest B gebunden steht
und die einzelnen, auch zweifach erscheinenden Formelglieder zueinander gleiche oder voneinander verschiedene Bedeutungen besitzen können,
zur Triphendioxazin-Verbindung entsprechend der allgemeinen Formel (2)

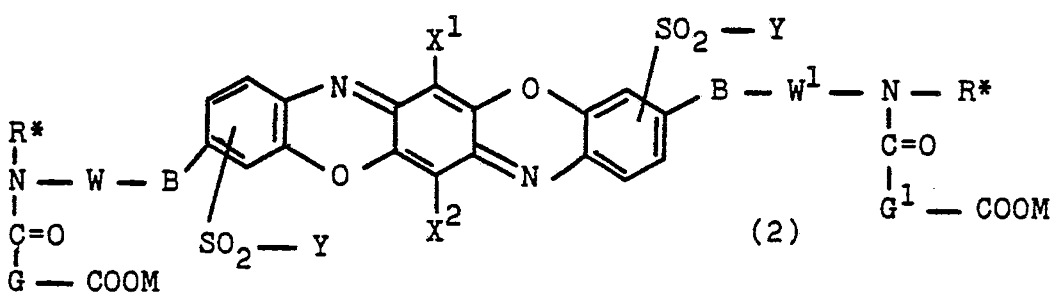
(2)

cyclisiert, in welcher B, G, $G^1$, $R^*$, W, $W^1$, $X^1$ und $X^2$ die obengenannten Bedeutungen haben und Y eine $\beta$-Sulfatoethyl-Gruppe oder eine Ethylgruppe ist, die in $\beta$-Stellung einen anderen alkalisch abspaltbaren Substituenten enthält.